(19) **Europäisches Patentamt / European Patent Office / Office européen des brevets**

(11) **EP 3 096 677 B1**

(12) **FASCICULE DE BREVET EUROPEEN**

(45) Date de publication et mention de la délivrance du brevet:
**05.08.2020 Bulletin 2020/32**

(21) Numéro de dépôt: **15704051.0**

(22) Date de dépôt: **15.01.2015**

(51) Int Cl.:
*A61B 3/028* (2006.01)    *A61B 3/036* (2006.01)

(86) Numéro de dépôt international:
**PCT/FR2015/050103**

(87) Numéro de publication internationale:
**WO 2015/107303 (23.07.2015 Gazette 2015/29)**

(54) **SYSTÈME DE COMPENSATION VISUELLE ET DISPOSITIF BINOCULAIRE D'OPTOMÉTRIE**

VISUELLES KOMPENSATIONSSYSTEM UND OPTOMETRISCHE BINOKULARE VORRICHTUNG

VISUAL COMPENSATION SYSTEM AND OPTOMETRIC BINOCULAR DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.01.2014 FR 1450433**

(43) Date de publication de la demande:
**30.11.2016 Bulletin 2016/48**

(73) Titulaire: **Essilor International**
**94220 Charenton-le-Pont (FR)**

(72) Inventeurs:
• **NAUCHE, Michel**
**94227 Charenton-le-Pont Cedex (FR)**
• **BOUTINON, Stéphane**
**94227 Charenton-le-Pont Cedex (FR)**

(74) Mandataire: **Jacobacci Coralis Harle**
**32, rue de l'Arcade**
**75008 Paris (FR)**

(56) Documents cités:
**EP-A1- 1 676 523      EP-A1- 2 034 338**
**WO-A1-2012/123549    US-A1- 2004 032 568**
**US-A1- 2009 153 796**

• **REN HONGWEN ET AL: "Variable-focus liquid lens by changing aperture", APPLIED PHYSICS LETTERS, A I P PUBLISHING LLC, US, vol. 86, no. 21, 17 May 2005 (2005-05-17), pages 211107-211107, XP012065605, ISSN: 0003-6951, DOI: 10.1063/1.1935749**

**Description**

DOMAINE TECHNIQUE AUQUEL SE RAPPORTE L'INVENTION

La présente invention concerne de manière générale le domaine de l'optométrie.

Elle concerne plus particulièrement un système de compensation visuelle et un dispositif binoculaire d'optométrie comprenant un tel système.

ARRIERE-PLAN TECHNOLOGIQUE

Dans le cadre de la mesure de l'acuité visuelle d'un patient, il a déjà été proposé de simuler la compensation visuelle à fournir, par exemple au moyen de lunettes d'essai ou d'un réfracteur, tel qu'une tête de réfraction.

Les lunettes d'essais peuvent accueillir successivement des verres d'essai ayant des corrections différentes, jusqu'à trouver la correction qui convient au patient.

Cette solution n'est pas pratique et nécessite le stockage séparé des verres d'essai dans des boîtes dédiées. Elle implique en outre des changements de lentilles qui provoquent des transitions de puissance de correction non désirées et non continues.

Dans la tête de réfraction, les verres d'essai sont placés sur plusieurs disques, entraînés en rotation manuellement ou à l'aide d'un mécanisme motorisé.

On comprend toutefois qu'un tel objet présente un encombrement et un poids importants, liés au nombre de verres placés sur chaque disque.

De plus, le champ de vision à travers la tête de réfraction est limité (effet *"tunnel"*) du fait de la pluralité de lentilles alignées afin d'obtenir diverses valeurs de correction.

Avant de présenter l'invention, on rappelle quelques définitions de notions utilisées dans l'exposé qui suit.

La puissance optique représente le degré auquel un élément optique peut faire converger ou diverger des rayons lumineux. Elle s'exprime en dioptrie et correspond à l'inverse de la focale en mètre.

On parle de puissance sphérique lorsque la puissance optique est la même dans tous les plans méridiens de la lentille (symétrie de révolution autour de l'axe optique).

Au contraire on parle d'astigmatisme lorsque la puissance optique varie en fonction des méridiens de la lentille. Dans le cas d'un élément optique présentant de l'astigmatisme, on parle de puissance cylindrique qui est la différence entre la puissance optique maximale selon un premier méridien et la puissance optique minimale selon un deuxième méridien. C'est le cas des surfaces toriques ou cylindriques.

Le document US 2004/032568 divulge un système optométrique avec une lentille Alvarez de puissance sphérique variable et deux lentilles de puissance cylindrique variable

OBJET DE L'INVENTION

Dans ce contexte, la présente invention propose un système de compensation visuelle permettant d'observer selon un axe optique d'observation avec une correction optique de puissance variable, caractérisé en ce qu'il comprend un premier élément optique mobile en rotation centrée sur l'axe optique et ayant une première puissance cylindrique selon l'axe optique, un second élément optique mobile en rotation centrée sur l'axe optique et ayant une seconde puissance cylindrique selon l'axe optique, et une lentille ayant pour axe ledit axe optique et de puissance sphérique variable.

Le premier élément optique et le second élément optique peuvent être mobiles en rotation indépendamment l'un de l'autre de sorte que, dans au moins une position, la puissance cylindrique résultante, générée par la combinaison du premier élément optique et du second élément optique, a une valeur négligeable, par exemple inférieure à 0,1 dioptrie, ou même une valeur nulle.

En pratique, la valeur absolue de la seconde puissance cylindrique est par exemple égale (ou au moins quasiment égale) à la valeur absolue de la première puissance cylindrique, de sorte que ladite valeur de puissance cylindrique résultante est nulle (ou quasi-nulle) dans au moins une position.

Autrement dit, dans ce cas, la seconde puissance cylindrique est égale ou opposée à la première puissance cylindrique. La première puissance cylindrique et la seconde puissance cylindrique peuvent toutefois être différentes afin de compenser l'espacement entre les deux lentilles (selon la formule de Gullstrand) de façon à obtenir un alignement dont la puissance cylindrique combinée (i.e. résultante) s'annule dans au moins une position.

Ainsi, en faisant varier la position angulaire du premier élément optique (angle $\alpha_1$ dans la description qui suit) et la position angulaire du second élément optique (angle $\alpha_2$ dans la description qui suit), indépendamment l'une de l'autre, ainsi que la puissance sphérique $S_V$ de la lentille à puissance sphérique variable, on peut faire varier indépendamment la puissance sphérique S, la puissance cylindrique C et l'angle d'astigmatisme $\alpha$ du système (formé par le premier élément optique, le second élément optique et la lentille à puissance sphérique variable) sur des plages prédéfinies, comme expliqué dans la description qui suit.

Notamment, grâce à la possibilité de faire varier l'orientation relative des deux éléments optiques de puissance cylindrique, il existe au moins une position du système pour laquelle la puissance cylindrique C du système est réduite. Lorsque la première puissance cylindrique et la seconde puissance cylindrique sont égales ou quasiment égales en valeur absolue, il existe au moins une position relative de ces deux éléments pour laquelle la puissance cylindrique C du système est négligeable, voire nulle. On est ainsi capable de générer une correction de puissance sphérique uniquement.

On remarque par ailleurs que la puissance sphérique variable permet notamment de compenser la puissance sphérique créée par l'association des éléments optiques de puissance cylindrique, soit pour l'annuler, soit pour obtenir au total (pour l'ensemble du système) une puissance sphérique conforme à la puissance sphérique souhaitée. Ainsi, la puissance sphérique induite par la combinaison du premier élément optique et du second élément optique peut être au moins en partie compensée par la lentille de puissance sphérique variable.

Ce système de compensation visuelle est ainsi particulièrement bien adapté pour générer des corrections variables ; il a de plus un encombrement réduit du fait que trois éléments optiques suffisent à réaliser des corrections variables dans les plages de paramètres précitées.

Ce système permet en outre de réaliser la fonction des cylindres croisés par retournement, par rotation rapide des deux éléments optiques à puissance cylindrique. Pour réaliser cette fonction (fréquemment utilisée dans les protocoles de réfraction), on utilise un bicylindre croisé composé de deux verres plan-cylindriques, d'axes perpendiculaires, de signes opposés et de puissances identiques. Sa puissance sphérique est nulle, on l'utilise pour faire varier très rapidement la valeur de la puissance cylindrique en retournant le bicylindre. Cette variation rapide est réalisable ici sans ajout d'éléments optiques supplémentaires, en entraînant en rotation de concert le premier élément optique et le second élément optique.

La lentille de puissance sphérique variable est par exemple une lentille déformable contenant un fluide, ou, autrement dit, une lentille contenant un fluide et une membrane déformable.

On peut prévoir un premier mécanisme entraîné par un premier moteur et conçu pour déplacer le premier élément optique en rotation centrée sur l'axe optique, ainsi qu'éventuellement un second mécanisme entraîné par un second moteur et conçu pour déplacer le second élément optique en rotation centrée sur l'axe optique.

Le premier mécanisme et le premier moteur d'une part, et le second mécanisme et le second moteur d'autre part, forment respectivement un premier actionneur et un second actionneur, qui permettent chacun le réglage en position de l'un des premier et second éléments optiques.

Le système de compensation visuelle peut comprendre un élément de commande conçu pour commander respectivement le premier moteur et le second moteur en fonction d'informations de consigne, par exemple des informations de consigne reçues d'une télécommande manipulée par un utilisateur du système.

L'élément de commande comprend par exemple un capteur de température et/ou un capteur d'orientation ou de mouvement conçu pour délivrer une information d'orientation.

On peut ainsi prévoir notamment que l'élément de commande comprenne un calculateur conçu pour générer des signaux de commande en fonction d'au moins une desdites informations de consigne et de ladite information d'orientation et pour émettre les signaux de commande respectivement à destination du premier moteur et du second moteur.

Les signaux de commande envoyés au moteur tiendront ainsi compte de l'orientation du système de compensation visuelle, par exemple afin de compenser les effets de puissance induite sur la lentille liquide dus à la gravité.

L'élément de commande peut également être conçu pour générer des signaux de commande en fonction d'au moins une desdites informations de consigne et d'une distance entre une partie du système et un œil observant à travers le système.

Le premier mécanisme peut comprendre une première roue dentée qui coopère par exemple avec une première vis sans fin solidaire d'un axe d'entraînement du premier moteur ; le premier élément optique peut alors être monté sur la première roue dentée.

De même, le second mécanisme peut comprendre une seconde roue dentée qui coopère par exemple avec une seconde vis sans fin solidaire d'un axe d'entraînement du second moteur ; le second élément optique peut alors être monté sur la seconde roue dentée.

De tels mécanismes permettent une démultiplication du mouvement en sortie du moteur. Le système de compensation visuelle a ainsi une résolution particulièrement fine et les paramètres S, C, $\alpha$, qui définissent la correction du système, peuvent ainsi prendre un ensemble quasiment continu de valeurs sur les plages précitées. De plus, grâce à de tels mécanismes, les roues dentées, et donc les éléments optiques portés par ces roues dentées, sont maintenus en position même en l'absence d'alimentation des moteurs. Les éléments optiques (c'est-à-dire le premier élément optique, le second élément optique et la lentille) sont ainsi montés (dans le système de compensation visuelle) de manière à conserver (chacun) leur position de consigne respective (même) sans alimentation électrique.

Le système de compensation visuelle peut comprendre au moins une cellule optique associée à l'une desdites roues dentées (en pratique une cellule optique associée à chaque roue dentée) de manière à déterminer la position de l'élément optique associé (parmi le premier élément optique, le second élément optique et la lentille).

Le système de compensation visuelle peut être logé dans un boîtier formé par exemple de l'assemblage d'au moins une première partie et une seconde partie ; on peut alors prévoir que la première roue dentée soit montée en rotation sur ladite première partie et que la seconde roue dentée soit montée en rotation sur ladite seconde partie.

Le premier moteur est par exemple monté sur ladite première partie et/ou le second moteur est par exemple monté sur ladite seconde partie.

On peut prévoir également qu'un troisième mécanisme entraîné par un troisième moteur soit conçu pour entraîner en rotation une bague de commande de la puissance sphérique de la lentille de puissance sphérique variable.

La puissance sphérique peut ainsi elle aussi être réglée au moyen d'un actionneur formé du troisième moteur et du troisième mécanisme.

Le troisième mécanisme comprend par exemple une troisième roue dentée qui coopère avec une troisième vis sans fin solidaire d'un axe d'entraînement du troisième moteur, la bague de commande étant solidaire de la troisième roue dentée.

Le premier moteur, le second moteur et le troisième moteur sont par exemple disposés de façon à libérer une géométrie circulaire sur au moins 120°, par exemple sur 180°, centrée sur l'axe optique au plus proche du rayon utile des lentilles, par exemple à une distance inférieure à 20 mm (voire inférieure à 10 mm) du rayon utile des lentilles ; on obtient ainsi un ensemble d'encombrement réduit.

L'élément de commande déjà mentionné (par exemple au moyen de son calculateur déjà mentionné) peut être conçu pour générer au moins un signal de commande, à destination du troisième moteur, en fonction d'au moins une desdites consignes et d'une information de température générée par le capteur de température. On peut ainsi compenser les variations de puissance sphérique de la lentille à puissance sphérique variable dues aux variations éventuelles de température.

Le boîtier peut par ailleurs comprendre une troisième partie, le troisième moteur pouvant alors être monté dans la troisième partie.

Selon des modes de réalisation envisageables (par exemple celui décrit ci-après), le premier élément optique est un premier dioptre formé sur une face d'une première lentille plan-cylindre et/ou le second élément optique est un second dioptre formé sur une face d'une seconde lentille plan-cylindre. Précisément, on peut prévoir que la première lentille est une lentille plan-cylindre convexe et/ou que la seconde lentille est une lentille plan-cylindre concave.

Par ailleurs, le premier élément optique, le second élément optique et la lentille peuvent être commandés de manière à réaliser une fonction de cylindres croisés par retournement, c'est-à-dire de sorte que la puissance cylindrique et/ou l'angle d'astigmatisme du système formé du premier élément optique, du second élément optique et de la lentille alterne(nt) (chacun) entre deux valeurs distinctes.

Autrement dit, l'invention propose un système de compensation visuelle permettant d'observer selon un axe optique d'observation avec une correction optique de puissance variable, caractérisé en ce qu'il comprend :

- un premier élément optique mobile en rotation centrée sur l'axe optique et ayant une première puissance cylindrique selon l'axe optique,
- un second élément optique mobile en rotation centrée sur l'axe optique et ayant une seconde puissance cylindrique selon l'axe optique,
- une lentille ayant pour axe ledit axe optique, de puissance sphérique variable et actionnable mécaniquement de manière à faire varier continûment ladite puissance sphérique.

L'invention propose également un dispositif binoculaire d'optométrie comprenant deux systèmes optiques, montés par exemple sur un support commun, dans lequel un des deux systèmes optiques (voire chacun des deux systèmes optiques) est un système de compensation visuelle comme présenté ci-dessus.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 représente schématiquement les éléments optiques utilisés dans un exemple de mise en œuvre de l'invention ;
- la figure 2 représente un vue en coupe d'un exemple de système de compensation visuelle conforme aux enseignements de l'invention ;

- la figure 3 représente une vue écorchée du système de compensation de la figure 2 côté lentilles cylindriques ;
- la figure 4 est une vue écorchée du système de compensation de la figure 2 côté lentille sphérique variable ;
- la figure 5 représente schématiquement un élément de commande du système de compensation visuelle de la figure 2.

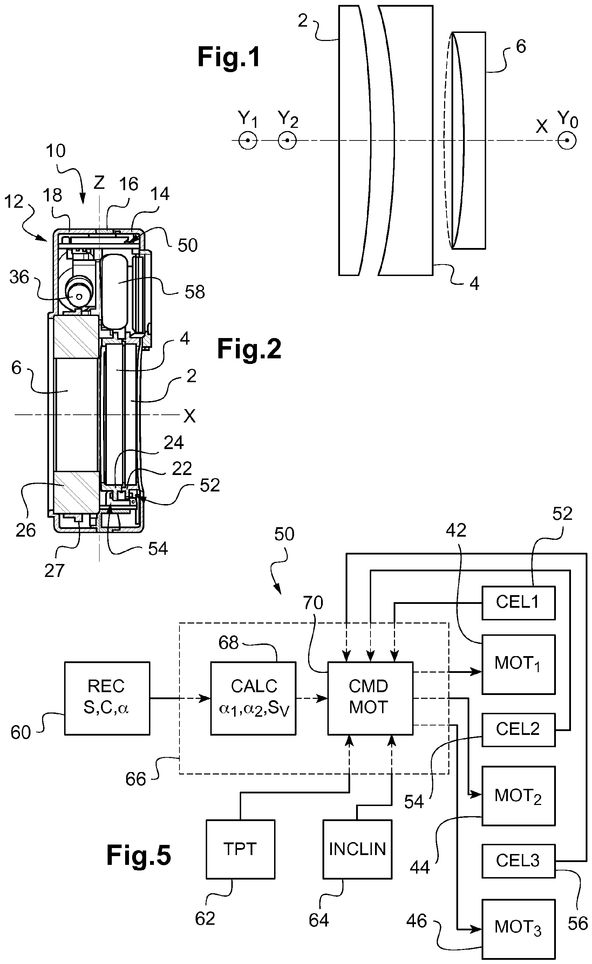

Sur la figure 1 sont schématiquement représentés les éléments optiques principaux d'un exemple de système de compensation visuelle conforme aux enseignements de l'invention.

Ces éléments optiques comprennent une lentille plan-cylindre convexe 2, de puissance cylindrique $C_0$, une lentille plan-cylindre concave 4, de puissance cylindrique négative $-C_0$, et une lentille 6 de puissance sphérique variable $S_V$.

La valeur absolue (ou module), ici $C_0$, de la puissance cylindrique (ici $-C_0$) de la lentille plan-cylindre concave 4 est donc égale à la valeur absolue ($C_0$) (ou module) de la puissance cylindrique ($C_0$) de la lentille plan-cylindre convexe 2.

Ces trois lentilles 2, 4, 6 sont placées sur le même axe optique X. Précisément, chacune des trois lentilles 2, 4, 6 a une forme extérieure généralement cylindrique, centrée sur l'axe optique X. Dans l'exemple décrit ici, les lentilles 2, 4, 6 ont respectivement les diamètres (mesurant leur encombrement) suivants : 25 mm, 25 mm, 20 mm.

On remarque de ce fait qu'il est préférable d'utiliser ce système de compensation visuelle 10 en positionnant l'œil du patient du côté de la lentille de puissance sphérique variable 6 de sorte que les lentilles de puissance cylindriques 2, 4, de plus grand diamètre, ne viennent pas limiter le champ de vision défini par la lentille de puissance sphérique variable 6, qui est lui-même large du fait de la proximité de l'œil du patient.

Chacune des trois lentilles 2, 4, 6 comporte une première face plane, perpendiculaire à l'axe optique X, et une seconde face, opposée à la première face et optiquement active :

- la face optiquement active de la lentille 2 est de forme cylindrique convexe (l'axe $Y_1$ du cylindre définissant cette face étant perpendiculaire à l'axe optique X) ;
- la face optiquement active de la lentille 4 est de forme cylindrique concave (l'axe $Y_2$ du cylindre définissant cette face étant perpendiculaire à l'axe optique X) ;
- la face optiquement active de la lentille 6 de puissance sphérique variable $S_V$ est déformable et peut ainsi prendre une forme sphérique convexe (comme illustré en pointillés sur la figure 1), une forme plane ou une forme sphérique concave (comme illustré en trait plein).

La lentille 6 de puissance sphérique variable $S_V$ est par exemple une lentille du type décrit dans le document EP 2 034 338. Une telle lentille comprend une cavité fermée par une membrane déformable transparente et une paroi plane transparente mobile ; la cavité contient un liquide transparent de volume constant qui est plus ou moins contraint par la face mobile, afin de déformer la membrane qui est de ce fait soit une surface concave sphérique, soit une surface plane, soit une surface convexe sphérique. Dans la lentille utilisée, une transformation de mouvement réalisée par un système vis écrou permet d'assurer la transformation de mouvement translation - rotation. Ainsi, une rotation d'une bague montée sur un boîtier 26 entraîne en translation une pièce de la lentille 6, ce qui provoque la déformation susmentionnée de la membrane transparente comme expliqué par exemple dans le document EP 2 034 338 précité. On peut ainsi faire varier continûment la puissance sphérique $S_V$ par action mécanique sur la lentille 6. Dans l'exemple décrit ici, la lentille 6 a une focale variable entre -40 mm et 40 mm, soit une puissance sphérique $S_V$ variable entre -25D et 25D (D étant la dioptrie, unité de mesure de la vergence, inverse de la focale exprimée en mètres).

Par ailleurs, les lentilles plan-cylindre 2, 4 ont respectivement comme déjà indiqué une puissance cylindrique $-C_0$ et $C_0$, ici avec $C_0 = 5D$.

Comme expliqué plus en détail dans la suite, la lentille plan-cylindre concave 4 et la lentille plan-cylindre convexe 2 sont montées en rotation autour de l'axe X (rotation centrée sur l'axe X).

L'axe $Y_1$ du cylindre convexe formé sur la face optiquement active de la lentille plan-cylindre convexe 2 peut ainsi former un angle variable $\alpha_1$ avec un axe de référence $Y_0$ (fixe et perpendiculaire à l'axe optique X).

De même, l'axe $Y_2$ du cylindre concave formé sur la face optiquement active de la lentille plan-cylindre concave 4 peut former un angle variable $\alpha_2$ avec l'axe de référence $Y_0$.

Par calcul de la vergence sur les différents méridiens, on obtient les formules suivantes pour la puissance sphérique S, la puissance cylindrique C et l'angle d'astigmatisme $\alpha$ du système formé des trois éléments optiques 2, 4, 6 qui vient d'être décrit :

$$\tan 2\alpha = \frac{\sin 2\alpha_2 - \sin 2\alpha_1}{\cos 2\alpha_2 - \cos 2\alpha_1} = -\frac{\cos(\alpha_1 + \alpha_2)}{\sin(\alpha_1 + \alpha_2)} \text{ (formule 1)}$$

$$C = C_0 \left( \cos 2(\alpha - \alpha_2) - \cos 2(\alpha - \alpha_1) \right) \text{ (formule 2)}$$

$$S = S_V - \frac{C}{2} \text{ (formule 3).}$$

On remarque que le terme (-C/2) dans la formule 3 correspond à puissance sphérique générée par la résultante des 2 lentilles à puissance cylindrique.

En pilotant la position en rotation de la lentille plan-cylindre convexe 2 et la position en rotation de la lentille plan-cylindre concave 4, indépendamment l'une de l'autre, comme décrit ci-après, on peut faire varier indépendamment chacun des angles $\alpha_1$, $\alpha_2$ de 0° à 360° et ainsi obtenir une puissance cylindrique C réglable entre -2.$C_0$ et 2.$C_0$ (soit ici entre -10D et 10D), et pour n'importe quel angle d'astigmatisme réglable entre 0° et 360° obtenue par une commande simultanée des deux lentilles. Comme l'indique la formule numéro 3, la résultante de puissance sphérique induite par la résultante de l'orientation des 2 lentilles cylindriques est compensée à l'aide de la lentille sphérique de puissance variable.

Par ailleurs, en faisant varier la puissance sphérique $S_V$ de la lentille sphérique 6, on peut régler la puissance sphérique S du système formé des trois lentilles 2, 4, 6.

Selon une variante envisageable, les lentilles à puissance cylindrique fixe pourraient avoir la même puissance cylindrique $C_0$ (positive ou négative) : il pourrait s'agir de deux lentilles plan-cylindre convexe, éventuellement identiques, ou, en alternative, de deux lentilles plan-cylindre concave, éventuellement identiques.

En effet, dans ce cas, la puissance sphérique S, la puissance cylindrique C et l'angle d'astigmatisme $\alpha$ du système formé de ces deux lentilles et d'une lentille à puissance sphérique variable sont donnés par les formules suivantes :

$$\tan 2\alpha = \frac{\sin 2\alpha_2 + \sin 2\alpha_1}{\cos 2\alpha_2 + \cos 2\alpha_1} \text{ (formule 4)}$$

$$C = C_0 \left( \cos 2(\alpha - \alpha_2) + \cos 2(\alpha - \alpha_1) \right) \text{ (formule 5)}$$

$$S = S_V + C_0 - \frac{C}{2} . \text{ (formule 6)}$$

Le terme $C_0$ - C/2 correspond à la puissance sphérique induite par la combinaison des deux lentilles à puissance cylindrique.

On peut donc également dans ce cas régler la puissance sphérique S, la puissance cylindrique C et l'angle d'astigmatisme $\alpha$, en particulier de sorte que la puissance cylindrique C soit nulle, en entraînant en rotation les lentilles à puissance cylindrique (indépendamment l'une de l'autre) et en faisant varier la puissance sphérique de la lentille à puissance sphérique variable.

Un exemple de système de compensation visuelle 10 qui utilise les éléments optiques qui viennent d'être décrits est représenté en figure 2.

On utilisera parfois dans la description qui suit, afin de clarifier l'explication, des termes, comme *"supérieur"* ou *"inférieur",* qui définissent une orientation dans les figures 2, 3 et 4. On comprend que cette orientation n'est pas nécessairement applicable à l'utilisation qui pourra être faite du système décrit, utilisation dont la seule direction de référence est l'axe optique X.

Le système de compensation visuelle 10 comprend un boîtier 12 formé d'une première partie 14, d'une seconde partie 16 et d'une troisième partie 18, qui s'étendent successivement selon l'axe optique X et sont assemblées deux à deux au niveau de plans perpendiculaires à l'axe optique X.

Une première roue dentée 22 est montée en rotation centrée sur l'axe optique X dans la première partie 14 du boîtier 12 et porte en son centre, dans une ouverture prévue à cet effet, la lentille plan-cylindre convexe 2. La première roue dentée 22 et la lentille plan-cylindre convexe 2 sont coaxiales ; autrement dit, en section dans un plan perpendiculaire à l'axe optique X, la circonférence extérieure de la première roue dentée 22 et la circonférence de la lentille plan-cylindre convexe 2 forment des cercles concentriques centrés sur l'axe optique X.

De même, une seconde roue dentée 24 est montée en rotation centrée sur l'axe optique X dans la seconde partie 16 du boîtier 12 et porte en son centre, dans une ouverture prévue à cet effet, la lentille plan-cylindre concave 4. La seconde roue dentée 24 et la lentille plan-cylindre concave 4 sont coaxiales ; autrement dit, en section dans un plan perpendiculaire à l'axe optique X, la circonférence extérieure de la seconde roue dentée 24 et la circonférence de la lentille plan-cylindre concave 4 forment des cercles concentriques centrés sur l'axe optique X.

Une troisième roue dentée 27 est montée en rotation centrée sur l'axe optique X dans la troisième partie 18 du boîtier 12. La troisième roue dentée 27 est solidaire de la bague pourvue sur la circonférence du boîtier 26 qui porte la lentille 6 de puissance sphérique variable et permettant la commande de la puissance sphérique $S_V$. Le boîtier 26 de la lentille 6 de puissance sphérique variable est monté dans la troisième partie 18 du boîtier 12.

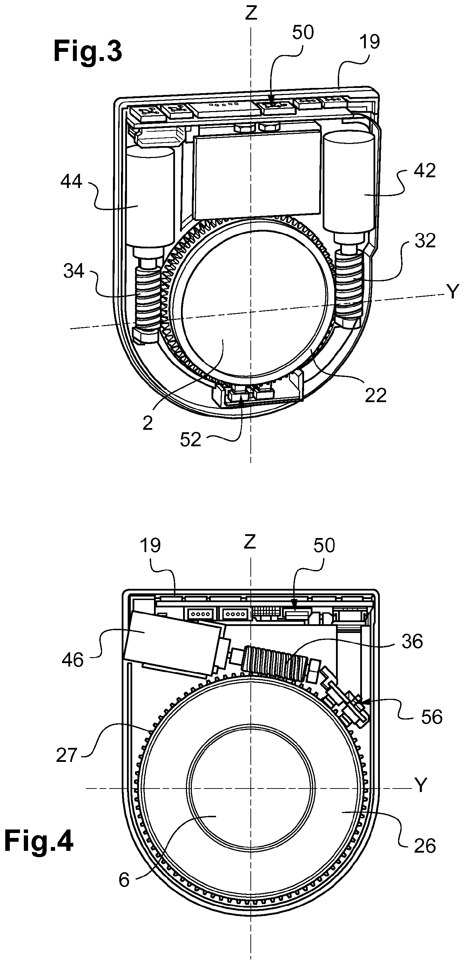

Comme bien visible en figure 3, la première roue dentée 22 est entraînée en rotation (autour de l'axe optique X) au moyen d'un premier moteur 42 dont un axe d'entraînement porte une première vis sans fin 32 qui engrène sur la première roue dentée 22. Le premier moteur 42 est par exemple monté dans la première partie 14 du boîtier 12.

La position courante de la première roue dentée 22 est surveillée par une première cellule optique 52.

De même, la seconde roue dentée 24 est entraînée en rotation autour de l'axe optique X au moyen d'un second moteur 44 dont un axe d'entraînement porte une seconde vis sans fin 34 qui engrène sur la seconde roue dentée 24. Le second moteur 44 est par exemple monté dans la seconde partie 16 du boîtier 12.

La position courante de la seconde roue dentée 24 est surveillée par une seconde cellule optique 54.

Comme représenté sur la figure 4, la troisième roue dentée 27 est quant à elle entraînée en rotation (autour de l'axe optique X) au moyen d'un troisième moteur 46 qui présente un axe d'entraînement sur lequel est monté une troisième vis sans fin 36 qui engrène avec la troisième roue dentée 27. Le troisième moteur 46 est par exemple monté dans la troisième partie 18 du boîtier 12.

La position courante de la troisième roue dentée 27 est surveillée par une troisième cellule optique 56.

Chaque cellule optique 52, 54, 56 est par exemple formée d'un couple d'éléments comprenant au moins un capteur optique ; l'autre élément du couple est par exemple un émetteur optique (ou, en variante, un élément réfléchissant, auquel cas un émetteur optique est associé au capteur optique).

Les premier, second et troisième moteurs 42, 44, 46 sont par exemple des moteurs pas à pas, d'une résolution de 20 pas/tour, pilotés ici en $8^{ème}$ de pas (ci-après micro-pas). En variante, ces moteurs pourraient être pilotés en $16^{ème}$ de pas.

Le volume interne du boîtier 12 (comme d'ailleurs le volume interne de chacune des première, seconde et troisième parties 14, 16, 18 de la même manière) peut être subdivisé en un espace de réception des moteurs 42, 44, 46 (région supérieure du boîtier 12 sur les figures 2, 3 et 4) et un espace de réception des éléments optiques 2, 4, 6 (région inférieure du boîtier 12 sur les figures 2, 3 et 4).

L'espace de réception des moteurs 42, 44, 46 a une forme essentiellement parallélépipédique, ouverte (vers le bas sur les figures) en direction de l'espace de réception des éléments optiques 2, 4, 6 et fermé à l'opposé (vers le haut sur les figures) par une face supérieure 19 du boîtier 12 (la face supérieure 19 du boîtier 12 étant formée par l'assemblage de faces supérieures respectives des première, seconde et troisième parties 14, 16, 18 du boîtier 12).

La disposition des moteurs 42 44 et 46 est telle qu'elle permet de bénéficier d'une géométrie circulaire sur 180° centrée sur l'axe optique au plus proche du rayon utile des lentilles.

L'espace de réception des éléments optiques 2, 4, 6 présente, à l'opposé de l'espace de réception des moteurs, une forme cylindrique (délimitée par les parois du boîtier 12) qui épouse celle de la troisième roue dentée 27 sur la moitié de la circonférence de celle-ci.

Autrement dit, le boîtier 12 (et par conséquent chacune des première, seconde et troisième parties 14, 16, 18 du boîtier 12) a, au niveau de l'espace de réception des éléments optiques 2, 4, 6, une forme cylindrique de diamètre (perpendiculairement à l'axe optique X) du même ordre que, et légèrement supérieur à, celui de la troisième roue dentée 27.

Les diamètres respectifs des roues dentées 22, 24, 27 sont adaptés de manière à favoriser la conservation du champ en dépit de l'épaisseur du système optique.

Le premier moteur 42 et la première vis sans fin 32 s'étendent dans le boîtier 12 selon une direction Z perpendiculaire à la face supérieure du boîtier 12 (et donc notamment perpendiculaire à l'axe optique X) de telle sorte que le premier moteur 42 est logé dans l'espace de réception des moteurs tandis que la première vis sans fin 32 s'étend dans l'espace de réception des éléments optiques.

Le second moteur 44 et la seconde vis sans fin 34 s'étendent quant à eux dans le boîtier 12 selon la même direction, mais à l'opposé du premier moteur 42 et de la première vis sans fin 34 par rapport aux lentilles de puissance cylindrique 2, 4. Le second moteur 44 est logé dans l'espace de réception des moteurs tandis que la seconde vis sans fin 34 s'étend dans l'espace de réception des éléments optiques.

On remarque qu'ainsi la première vis sans fin 32 et la seconde vis sans fin 34 sont situées de part et d'autre de l'ensemble formé par la première roue dentée 22 et la seconde roue dentée 24, et que l'encombrement latéral (selon un axe Y perpendiculaire aux axes X et Z précités) de ces différentes pièces (première vis sans fin 32, seconde vis sans fin 34, première ou seconde roue dentée 22, 24) est inférieur au diamètre de la troisième roue dentée 27 de sorte que les première et seconde vis sans fin 32, 34 contiennent dans l'espace de réception des éléments optiques sans nécessiter d'excroissance pour les accueillir.

Par ailleurs, les premier et second moteurs 42, 44 ont chacun un encombrement selon l'axe optique X supérieur à celui de chacune des première et seconde roues dentées 22, 24, et même supérieur à celui de chacune des première et seconde parties 14, 16 de boîtier 12. Toutefois, du fait que ces premier et second moteurs 42, 44 sont placés comme il vient d'être indiqué de chaque côté du boîtier 12 (par rapport à l'axe Z), ils peuvent chacun occuper un espace qui s'étend selon l'axe optique X au droit de la première partie 14 et de la seconde partie 16 du boîtier 12.

Par exemple, chacun des premier et second moteurs 42, 44 a un encombrement latéral (diamètre externe du moteur) compris entre 6 et 12, par exemple 10 mm, tandis que les première et seconde roues dentées 22, 24 ont chacune une épaisseur (encombrement selon l'axe X) compris entre 1 et 4, par exemple 2,5 mm.

Le troisième moteur 46 et la troisième vis sans fin 36 sont en revanche situés dans l'espace de réception des moteurs, dans la région qui s'étend selon l'axe X au droit de la troisième partie 18 du boîtier 12. Ainsi, la troisième vis sans fin 36 engrène la troisième roue dentée 27 dans une partie supérieure de celle-ci, ce qui permet au boîtier 12 d'épouser la forme du boîtier 12 dans la partie inférieure de la troisième roue dentée 27, comme déjà indiqué.

Dans l'exemple décrit, comme visible en figure 4, l'axe du troisième moteur 46 et de la troisième vis sans fin 36 est légèrement incliné par rapport à la face supérieure du boîtier 12 (précisément par rapport à l'axe Y précité).

On prévoit par exemple que l'épaisseur de la troisième roue dentée 27 est comprise entre 0,3 mm et 2 mm.

Cette disposition des différents éléments permet d'obtenir un boîtier relativement fin, ayant typiquement une épaisseur comprise entre 15 et 20 mm.

Le boîtier 12 comprend également, par exemple dans la région supérieure de l'espace de réception des moteurs, un élément de commande 50, formé ici de plusieurs circuits intégrés portés par un circuit imprimé commun.

Par ailleurs un dispositif de stockage d'énergie électrique de type batterie 58 (ou, en variante, une super capacité) est prévu pour rendre l'appareil autonome. On prévoit par exemple également des éléments de recharge sans contact du dispositif de stockage d'énergie 58. La batterie 58 permet notamment l'alimentation électrique des moteurs 42, 44, 46 et de l'élément de commande 50.

Les éléments principaux d'un tel élément de commande 50, ainsi que leur connexion aux moteurs 42, 44, 46 précités et aux cellules optiques 52, 54, 56 précitées, sont représentés schématiquement en figure 5.

L'élément de commande 50 comprend un module de réception 60 conçu pour recevoir, ici à travers une liaison sans fil, les informations de consigne, c'est-à-dire des informations indicatives des valeurs souhaitées par l'utilisateur pour la puissance sphérique S, la puissance cylindrique C et l'angle d'astigmatisme $\alpha$ qui définissent la compensation générée par le système optique formé des éléments optiques 2, 4, 6.

Le module de réception 60 est par exemple un module de réception infrarouge qui reçoit ces informations de consigne d'une télécommande à émission infrarouge manipulée par l'utilisateur. En variante, on pourrait prévoir que ces informations de consigne soit reçues d'un ordinateur personnel via une liaison sans fil, par exemple un réseau local sans fil ; l'utilisateur pourrait dans ce cas choisir des valeurs de puissance sphérique S, de puissance cylindrique C et d'angle d'astigmatisme $\alpha$ pour le système de compensation visuelle par sélection interactive sur l'ordinateur.

Le module de réception 60 transmet les informations de consigne S, C, $\alpha$ reçues à un calculateur 66 (constitué par exemple d'un processeur exécutant un programme d'ordinateur de manière à mettre en œuvre les fonctions du calculateur décrites ci-après), précisément à un module de calcul 68 mis en œuvre par ce calculateur 66.

Le module de calcul 68 calcule les valeurs des angles $\alpha_1$, $\alpha_2$ et la valeur de puissance sphérique $S_V$ nécessaires afin d'obtenir les valeurs de consignes S, C, $\alpha$ reçues en entrée, sur la base des formules exposées plus haut. Dans le cas où les lentilles plan-cylindre 2 et 4 ont respectivement une puissance cylindrique - $C_0$ et $C_0$, on utilise par exemple les formules suivantes :

$$\begin{cases} \alpha_1 = \alpha - \dfrac{1}{2}\arcsin\left(\dfrac{C}{2C_0}\right) + \dfrac{\pi}{4} \\[2em] \alpha_2 = \alpha + \dfrac{1}{2}\arcsin\left(\dfrac{C}{2C_0}\right) + \dfrac{\pi}{4} \end{cases}$$

$$S_V = S + \frac{C}{2}$$

Le calculateur 66 met également en œuvre un module de commande 70 qui reçoit en entrée les valeurs d'angle $\alpha_1$, $\alpha_2$ et de puissance sphérique $S_V$ calculées par le module de calcul 68 et émet des signaux de commande à destination des moteurs 42, 44, 46 afin de commander chacun des moteurs 42, 44, 46 indépendamment des autres de manière à obtenir des positionnements respectifs des roues dentées 22, 24, 27 qui permettent d'obtenir les valeurs souhaitées :

- le module de commande 70 commande le premier moteur 42 de manière à faire tourner la première roue dentée 22 autour de l'axe optique X jusqu'à la position où l'axe $Y_1$ de la surface cylindrique optiquement active de la lentille plan-cylindre convexe 2 (portée par la première roue dentée 22) forme un angle $\alpha_1$ avec la direction de référence $Y_0$ ;
- le module de commande 70 commande le second moteur 44 de manière à faire tourner la seconde roue dentée 24 autour de l'axe optique X jusqu'à la position où l'axe $Y_2$ de la surface cylindrique optiquement active de la lentille plan-cylindre concave 4 (portée par la seconde roue dentée 24) forme un angle $\alpha_2$ avec la direction de référence $Y_0$ ;
- le module de commande 70 commande le troisième moteur 46 de manière à faire tourner la troisième roue dentée 27 autour de l'axe optique X jusqu'à la position où la bague de commande de la puissance sphérique variable commande la puissance sphérique $S_V$ calculée par le module de calcul 68.

La position de chaque roue dentée 22, 24, 27 est connue à chaque instant respectivement grâce aux cellules optiques 52, 54, 56 qui mesurent chacune, sur la roue dentée à laquelle chacune est associée, le nombre de dents ayant traversé la cellule optique par rapport à un point de référence sur la circonférence de la roue concernée (par exemple dépourvu de dent).

Dans l'exemple décrit ici, l'ensemble premier moteur 42-première vis sans fin 32-première roue dentée 22, comme l'ensemble second moteur 44-seconde vis sans fin 34-seconde roue dentée 24, génère une démultiplication telle qu'un tour de roue dentée 22, 24 correspond à 15040 micro-pas du moteur associé 42, 44. La résolution (angle de rotation des roues dentées 22, 24 pour un micro-pas) est donc de 0,024° pour les angles $\alpha_1$ et $\alpha_2$.

L'ensemble troisième moteur 46-troisième vis sans fin 36-troisième roue dentée 46 génère quant à lui une démultiplication de 16640 micro-pas par tour. La bague de commande de la puissance sphérique variable est réglable sur une plage angulaire de 120° (ce qui correspond donc à 5547 micro-pas) afin d'obtenir la variation de puissance sphérique de -25D à 25D (soit une plage de variation de 50D). La résolution (variation de puissance sphérique $S_V$ pour un micro-pas) est donc de 0,009D.

Selon un mode de réalisation envisageable, on peut prévoir que l'élément de commande 50 prenne en compte la distance entre la face d'entrée de la lentille sphérique 6 et le sommet de la cornée d'un œil observant à travers le système de compensation visuelle, afin de corriger éventuellement les consignes en puissance du dispositif de compensation visuelle concerné. Cette distance (parfois dénommée DVO, pour *"distance verre-œil"*) peut être obtenue par des moyens connus pour ce faire.

En prenant l'exemple d'une puissance sphérique S de focale équivalente F, une erreur de positionnement $\varepsilon$ revient à avoir une correction de focale F', équivalente à une puissance sphérique S', avec :

$$F' = F - \varepsilon \quad \text{et} \quad S' = S\left( \frac{1}{1 - \dfrac{\varepsilon}{F}} \right),$$

ce qui donne en première approximation $S' = S \cdot (1 + \varepsilon \cdot S)$.

L'élément de commande 50 détermine donc, selon ce mode de réalisation, les valeurs des angles $\alpha_1$, $\alpha_2$ et la valeur de puissance sphérique $S_V$ (et les signaux de commandes à appliquer respectivement aux moteurs comme indiqué ci-dessus) non seulement en fonction des valeurs de consignes S, C, $\alpha$ reçues en entrée mais également en fonction de la distance œil - dispositif (ici cornée - face d'entrée de la lentille 6). On remarque que la distance verre-œil est ici prise en compte par l'élément de commande 50, qui reçoit les consignes brutes (c'est-à-dire sans prise en compte de la distance verre-œil).

Par ailleurs, on peut prévoir que, lors du passage de valeurs de consigne initiales $\alpha_1$, $\alpha_2$, Sv à de nouvelles valeurs de consigne $\alpha'_1$, $\alpha'_2$, $S'_V$, chacun des premier, second et troisième moteurs 42, 44, 46 soient actionnés pendant une même durée T (en secondes), qui peut dépendre éventuellement de l'amplitude de l'un des changements de consigne (par exemple de la variation, en valeur absolue, de puissance sphérique $|S'_V - S_V|$, où |x| est la valeur absolue de x).

Pour ce faire, le calculateur 66 détermine par exemple le nombre $p_1$ de micro-pas du moteur 42 permettant le passage de l'angle $\alpha_1$ à l'angle $\alpha'_1$, le nombre $p_2$ de micro-pas du moteur 44 permettant le passage de l'angle $\alpha_2$ à l'angle $\alpha'_2$ et le nombre $p_3$ de micro-pas du moteur 46 permettant le passage de la puissance sphérique $S_V$ à la puissance sphérique $S'_V$. Le calculateur 66 commande alors la rotation du moteur 42 à une vitesse de $p_1/T$ micro-pas par seconde, la rotation du moteur 44 à une vitesse de $p_2/T$ micro-pas par seconde et la rotation du moteur 46 à une vitesse de $p_3/T$

micro-pas par seconde.

L'élément de commande 50 comprend également un capteur de température 62, qui délivre une information de température ambiante mesurée, et un inclinomètre 64, par exemple réalisé sous forme d'un accéléromètre et qui délivre une information d'orientation du système de compensation visuelle 10, par exemple par rapport à la verticale.

Le calculateur 66 reçoit l'information de température en provenance du capteur de température 62 et l'information d'orientation en provenance de l'inclinomètre 64 et utilise ces informations dans le cadre de la détermination des commandes à envoyer aux moteurs 42, 44, 46.

Dans l'exemple décrit, le module de commande 70 utilise l'information de température afin de compenser les variations de puissance sphérique de la lentille 6 dues à la température (qui sont de l'ordre de 0,06D/°C dans l'exemple décrit) et l'information d'orientation afin de compenser les perturbations éventuelles du système d'entraînement (moteurs, vis sans fin, roues dentées) dues à des changements d'orientation du système de compensation visuelle 10.

Le système de compensation visuelle 10 peut être utilisé pour réaliser la fonction des cylindres croisés par retournement, également appelés cylindres de Jackson.

Selon un premier exemple, cette fonction peut être utilisée pour vérifier (voire trouver) un angle $\alpha_0$ de correction cylindrique requise (paramètre parfois dénommé *"axe du cylindre"*). On considère ici qu'une valeur de puissance de correction sphérique $S_0$ et une valeur de puissance de correction cylindrique $C_0$ ont été également déterminées au préalable.

La fonction des cylindres croisés par retournement est alors par exemple réalisée en appliquant en alternance rapide deux ensembles de consignes, soit un premier ensemble de consignes correspondant à un ajoût de puissance cylindrique $C_{var}$ (par exemple 0,5D) à 45° de l'axe défini par l'angle $\alpha_0$ :

- une consigne d'angle d'astigmatisme $\alpha_1 = \alpha_0 + 0,5.atan(C_{var}/C_0)$ ;
- une consigne de puissance cylindrique $C_1 = Racine(C_0^2+C_{var}^2)$, où Racine est la fonction racine carrée ;
- une consigne de puissance sphérique $S_1 = S_0 + C_0/2 - C_1/2$,

et un second ensemble de consignes correspondant à un ajoût de puissance cylindrique $-C_{var}$ à 45° de l'axe défini par l'angle $\alpha_0$ :

- une consigne d'angle d'astigmatisme $\alpha_2 = \alpha_0 - 0,5.atan(C_{var}/C_0)$ ;
- une consigne de puissance cylindrique $C_2 = Racine(C_0^2+C_{var}^2)$ ;
- une consigne de puissance sphérique $S_2 = S_0 + C_0/2 - C_2/2$.

Selon un second exemple, cette fonction peut être utilisée pour vérifier (voire trouver) la valeur de la puissance de correction cylindrique $C_0$ requise. On considère ici qu'une valeur de puissance de correction sphérique $S_0$ et une valeur d'angle d'astigmatisme $\alpha_0$ ont été également déterminées au préalable.

La fonction des cylindres croisés par retournement est alors par exemple réalisée en appliquant en alternance rapide deux ensembles de consignes, soit un premier ensemble de consignes correspondant à un ajoût de puissance cylindrique $C_{var}$ (par exemple 0,5D) dans l'axe défini par l'angle $\alpha_0$ :

- une consigne d'angle d'astigmatisme $\alpha_1 = \alpha_0$ ;
- une consigne de puissance cylindrique $C_1 = C_0 + C_{var}$ ;
- une consigne de puissance sphérique $S_1 = S_0 - C_{var}/2$,

et un second ensemble de consignes correspondant à un ajoût de puissance cylindrique $-C_{var}$ dans l'axe défini par l'angle $\alpha_0$ :

- une consigne d'angle d'astigmatisme $\alpha_2 = \alpha_0$ ;
- une consigne de puissance cylindrique $C_2 = C_0 - C_{var}$ ;
- une consigne de puissance sphérique $S_2 = S_0 + C_{var}/2$.

**Revendications**

**1.** Système de compensation visuelle (10) permettant d'observer selon un axe optique (X) d'observation avec une correction optique de puissance variable, comprenant :

- un premier élément optique (2) mobile en rotation centrée sur l'axe optique (X) et ayant une première puissance cylindrique selon l'axe optique (X),

- un second élément optique (4) mobile en rotation centrée sur l'axe optique (X) et ayant une seconde puissance cylindrique selon l'axe optique (X),
- une lentille (6) ayant pour axe ledit axe optique et de puissance sphérique variable,

**caractérisé en ce qu'**il comprend un mécanisme (36, 27) entraîné par un moteur (46) conçu pour entraîner en rotation une bague de commande de la puissance sphérique de la lentille de puissance sphérique variable (6).

2. Système de compensation visuelle selon la revendication 1, dans lequel le premier élément optique (2) et le second élément optique (4) sont mobiles en rotation indépendamment l'un de l'autre de sorte que, dans au moins une position, la puissance cylindrique résultante, générée par la combinaison du premier élément optique (2) et du second élément optique (4), a une valeur inférieure à 0,1 dioptrie.

3. Système de compensation visuelle selon la revendication 1 ou 2, dans lequel le premier élément optique (2) et le second élément optique (4) sont mobiles en rotation indépendamment l'un de l'autre de sorte que, dans au moins une position, la puissance cylindrique résultante, générée par la combinaison du premier élément optique (2) et du second élément optique (4), a une valeur nulle.

4. Système de compensation visuelle selon l'une des revendications 1 à 3, dans lequel la puissance sphérique induite par la combinaison du premier élément optique (2) et du second élément optique (4) est au moins en partie compensée par la lentille (6) de puissance sphérique variable.

5. Système de compensation visuelle selon l'une des revendications 1 à 4, dans lequel la lentille (6) de puissance sphérique variable est une lentille contenant un fluide et une membrane déformable.

6. Système de compensation visuelle selon l'une des revendications 1 à 4, dans lequel la lentille (6) de puissance sphérique variable est une lentille déformable contenant un fluide.

7. Système de compensation visuelle selon l'une des revendications 1 à 6, dans lequel un premier mécanisme (32, 22) entraîné par un premier moteur (42) est conçu pour déplacer le premier élément optique (2) en rotation centrée sur l'axe optique (X) et dans lequel un second mécanisme (34, 24) entraîné par un second moteur (44) est conçu pour déplacer le second élément optique (4) en rotation centrée sur l'axe optique (X).

8. Système de compensation visuelle selon la revendication 7, dans lequel un élément de commande (50) est conçu pour commander respectivement le premier moteur (42) et le second moteur (44) en fonction d'informations de consigne.

9. Système de compensation visuelle selon la revendication 8, dans lequel l'élément de commande (50) comprend un capteur de température (62).

10. Système de compensation visuelle selon la revendication 8 ou 9, dans lequel l'élément de commande (50) comprend un capteur (64) d'orientation ou de mouvement conçu pour délivrer une information d'orientation.

11. Système de compensation visuelle selon la revendication 10, dans lequel l'élément de commande (50) comprend un calculateur (66) conçu pour générer des signaux de commande en fonction d'au moins une desdites informations de consigne et de ladite information d'orientation et pour émettre les signaux de commande respectivement à destination du premier moteur (42) et du second moteur (44).

12. Système de compensation visuelle selon l'une des revendications 8 à 11, dans lequel l'élément de commande (50) est conçu pour générer des signaux de commande en fonction d'au moins une desdites informations de consigne et d'une distance entre une partie du système et un œil observant à travers le système.

13. Système de compensation visuelle selon l'une des revendications 7 à 12, dans lequel le premier mécanisme comprend une première roue dentée (22) qui coopère avec une première vis sans fin (32) solidaire d'un axe d'entraînement du premier moteur (42), le premier élément optique (2) étant monté sur la première roue dentée (22), et dans lequel le second mécanisme comprend une seconde roue dentée (24) qui coopère avec une seconde vis sans fin (34) solidaire d'un axe d'entraînement du second moteur (44), le second élément optique (4) étant monté sur la seconde roue dentée (24).

14. Système de compensation visuelle selon l'une des revendications 7 à 13, dans lequel le mécanisme conçu pour entraîner en rotation la bague de commande comprend une troisième roue dentée (27) qui coopère avec une troisième vis sans fin (36) solidaire d'un axe d'entrainement dudit moteur (46), la bague de commande étant solidaire de la troisième roue dentée (27).

15. Système de compensation visuelle selon la revendication 14 prise dans la dépendance de la revendication 13, dans lequel le premier moteur (42), le second moteur (44) et ledit moteur (46) sont disposés de façon à libérer une géométrie circulaire sur au moins 120° centrée sur l'axe optique (X) à une distance inférieure à 20 mm du rayon utile des lentilles.

16. Système de compensation visuelle selon l'une des revendications 13 à 15, comprenant au moins une cellule optique (52 ; 54 ; 56) associée à l'une desdites roues dentées (22 ; 24 ; 27) de manière à déterminer la position de l'élément optique associé (2 ; 4 ; 6).

17. Système de compensation visuelle selon l'une des revendications 1 à 16, dans lequel le premier élément optique, le second élément optique et la lentille sont montés de manière à conserver leur position de consigne respective sans alimentation électrique.

18. Système de compensation visuelle selon l'une des revendications 1 à 17, dans lequel le premier élément optique (2) et le second élément optique (4) sont séparés par un espace de dimension inférieure à 1 mm selon l'axe optique (X).

19. Système de compensation visuelle selon l'une des revendications 1 à 18, dans lequel le premier élément optique (2) est un premier dioptre formé sur une face d'une première lentille plan-cylindre et dans lequel le second élément optique (4) est un second dioptre formé sur une face d'une seconde lentille plan-cylindre.

20. Système de compensation visuelle selon la revendication 19, dans lequel le premier élément optique (2) est une lentille plan-cylindre convexe et dans lequel le second élément optique (4) est une lentille plan-cylindre concave.

21. Système de compensation visuelle selon l'une des revendications 1 à 20, dans lequel le premier élément optique (2), le second élément optique (4) et la lentille (6) sont commandés de manière à réaliser une fonction de cylindres croisés par retournement.

22. Système de compensation visuelle selon l'une des revendications 1 à 21, dans lequel la lentille (6) de puissance sphérique variable est actionnable mécaniquement de manière à faire varier continûment ladite puissance sphérique.

23. Dispositif binoculaire d'optométrie comprenant deux systèmes optiques, dans lequel au moins un des deux systèmes optiques est un système de compensation visuelle conforme à l'une des revendications 1 à 22.

**Patentansprüche**

1. System (10) für visuelle Kompensation, das ermöglicht, längs einer optischen Beobachtungsachse (X) mit einer variablen optischen Leistungskorrektur zu beobachten, das Folgendes umfasst:

   - ein erstes optisches Element (2), das rotatorisch beweglich ist, auf die optische Achse (X) zentriert ist und eine erste Zylinderleistung längs der optischen Achse (X) besitzt,
   - ein zweites optisches Element (4), das rotatorisch beweglich ist, auf die optische Achse (X) zentriert ist und eine zweite Zylinderleistung längs der optischen Achse (X) besitzt,
   - eine Linse (6), die als Achse die optische Achse besitzt und eine variable sphärische Leistung besitzt,

   **dadurch gekennzeichnet, dass** es einen Mechanismus (36, 27) enthält, der durch einen Motor (46) angetrieben wird, der dafür ausgelegt ist, einen Steuerring der sphärischen Leistung der Linse (6) mit variabler sphärischer Leistung rotatorisch anzutreiben.

2. System für visuelle Kompensation nach Anspruch 1, wobei das erste optische Element (2) und das zweite optische Element (4) unabhängig voneinander rotatorisch beweglich sind, derart, dass an wenigstens einer Position die resultierende zylindrische Leistung, die durch die Kombination des ersten optischen Elements (2) und des zweiten optischen Elements (4) erzeugt wird, einen Wert kleiner als 0,1 Dioptrien hat.

**3.** System für visuelle Kompensation nach Anspruch 1 oder 2, wobei das erste optische Element (2) und das zweite optische Element (4) unabhängig voneinander rotatorisch beweglich sind, derart, dass an wenigstens einer Position die resultierende zylindrische Leistung, die durch die Kombination des ersten optischen Elements (2) und des zweiten optischen Elements (4) erzeugt wird, den Wert null hat.

**4.** System für visuelle Kompensation nach einem der Ansprüche 1 bis 3, wobei die durch die Kombination des ersten optischen Elements (2) und des zweiten optischen Elements (4) induzierte sphärische Leistung wenigstens teilweise durch die Linse (6) mit variabler sphärischer Leistung kompensiert wird.

**5.** System für visuelle Kompensation nach einem der Ansprüche 1 bis 4, wobei die Linse (6) mit variabler sphärischer Leistung eine Linse ist, die ein Fluid und eine verformbare Membran enthält.

**6.** System für visuelle Kompensation nach einem der Ansprüche 1 bis 4, wobei die Linse (6) mit variabler sphärischer Leistung eine verformbare Linse ist, die ein Fluid enthält.

**7.** System für visuelle Kompensation nach einem der Ansprüche 1 bis 6, wobei ein erster Mechanismus (32, 22), der durch einen ersten Motor (42) angetrieben wird, dafür ausgelegt ist, das erste optische Element (2) mit der optischen Achse (X) als Zentrum rotatorisch zu verlagern, und wobei ein zweiter Mechanismus (34, 24), der durch einen zweiten Motor (44) angetrieben wird, dafür ausgelegt ist, das zweite optische Element (4) mit der optischen Achse (X) als Zentrum rotatorisch zu verlagern.

**8.** System für visuelle Kompensation nach Anspruch 7, wobei ein Steuerelement (50) dafür ausgelegt ist, den ersten Motor (42) bzw. den zweiten Motor (44) als Funktion von Sollwertinformationen zu steuern.

**9.** System für visuelle Kompensation nach Anspruch 8, wobei das Steuerelement (50) einen Temperatursensor (62) enthält.

**10.** System für visuelle Kompensation nach Anspruch 8 oder 9, wobei das Steuerelement (50) einen Orientierungs- oder Bewegungssensor (64) enthält, der dafür ausgelegt ist, Orientierungsinformationen zu liefern.

**11.** System für visuelle Kompensation nach Anspruch 10, wobei das Steuerelement (50) einen Rechner (66) enthält, der dafür ausgelegt ist, Steuersignale als Funktion der Sollwertinformationen und/oder der Orientierungsinformationen zu erzeugen und die Steuersignale zu dem ersten Motor (42) bzw. zu dem zweiten Motor (44) zu senden.

**12.** System für visuelle Kompensation nach einem der Ansprüche 8 bis 11, wobei das Steuerelement (50) dafür ausgelegt ist, Steuersignale als Funktion der Sollwertinformationen und/oder eines Abstands zwischen einem Teil des Systems und einem Auge, das durch das System hindurch beobachtet, zu erzeugen.

**13.** System für visuelle Kompensation nach einem der Ansprüche 7 bis 12, wobei der erste Mechanismus ein erstes Zahnrad (22) umfasst, das mit einer ersten Endlosschraube (32) zusammenwirkt, die mit einer Antriebsachse des ersten Motors (42) fest verbunden ist, wobei das erste optische Element (2) an dem ersten Zahnrad (22) montiert ist, und wobei der zweite Mechanismus ein zweites Zahnrad (24) enthält, das mit einer zweiten Endlosschraube (34) zusammenwirkt, die mit einer Antriebsachse des zweiten Motors (44) fest verbunden ist, wobei das zweite optische Element (4) an dem zweiten Zahnrad (24) montiert ist.

**14.** System für visuelle Kompensation nach einem der Ansprüche 7 bis 13, wobei der Mechanismus, der dafür ausgelegt ist, den Steuerring rotatorisch anzutreiben, ein drittes Zahnrad (27) enthält, das mit einer dritten Endlosschraube (36) zusammenwirkt, die mit einer Antriebsachse des Motors (46) fest verbunden ist, wobei der Steuerring mit dem dritten Zahnrad (27) fest verbunden ist.

**15.** System für visuelle Kompensation nach Anspruch 14, wenn abhängig von Anspruch 13, wobei der erste Motor (42), der zweite Motor (44) und der Motor (46) in der Weise angeordnet sind, dass über wenigstens 120° mit der optischen Achse (X) als Zentrum in einem Abstand von weniger als 20 mm des Nutzradius der Linsen eine kreisförmige Geometrie freigegeben wird.

**16.** System für visuelle Kompensation nach einem der Ansprüche 13 bis 15, das wenigstens eine optische Zelle (52;

54; 56) enthält, die einem der Zahnräder (22; 24; 27) zugeordnet ist, derart, dass die Position des zugeordneten optischen Elements (2; 4; 6) bestimmt wird.

17. System für visuelle Kompensation nach einem der Ansprüche 1 bis 16, wobei das erste optische Element, das zweite optische Element und die Linse in der Weise montiert sind, dass ihre jeweilige Sollposition ohne elektrische Versorgung beibehalten wird.

18. System für visuelle Kompensation nach einem der Ansprüche 1 bis 17, wobei das erste optische Element (2) und das zweite optische Element (4) durch einen Zwischenraum mit einer Abmessung von weniger als 1 mm längs der optischen Achse (X) getrennt sind.

19. System für visuelle Kompensation nach einem der Ansprüche 1 bis 18, wobei das erste optische Element (2) ein erster Diopter ist, der auf einer Fläche einer ersten planzylindrischen Linse gebildet ist, und wobei das zweite optische Element (4) ein zweiter Diopter ist, der auf einer Fläche einer zweiten planzylindrischen Linse gebildet ist.

20. System für visuelle Kompensation nach Anspruch 19, wobei das erste optische Element (2) eine konvexe planzylindrische Linse ist und wobei das zweite optische Element (4) eine konkave planzylindrische Linse ist.

21. System für visuelle Kompensation nach einem der Ansprüche 1 bis 20, wobei das erste optische Element (2), das zweite optische Element (4) und die Linse (6) in der Weise gesteuert werden, dass eine Funktion gekreuzter Zylinder durch Umdrehen verwirklicht wird.

22. System für visuelle Kompensation nach einem der Ansprüche 1 bis 21, wobei die Linse (6) mit variabler sphärischer Leistung mechanisch betätigbar ist, derart, dass die sphärische Leistung kontinuierlich variiert wird.

23. Optometrische binokulare Vorrichtung, die zwei optische Systeme umfasst, wobei wenigstens eines der beiden optischen Systeme ein System für visuelle Kompensation nach einem der Ansprüche 1 bis 22 ist.


**Claims**

1. A visual compensation system (10) allowing observation along an optical axis (X) of observation with an optical correction of variable power, comprising:

   - a first optical element (2) rotatable with a rotary movement centred on the optical axis (X) and having a first cylindrical power along the optical axis (X);
   - a second optical element (4) rotatable with a rotary movement centred on the optical axis (X) and having a second cylindrical power along the optical axis (X);
   - a lens (6) having for axis said optical axis and of variable spherical power,

   **characterised in that** it comprises a mechanism (36, 27) driven by a motor (46) designed to drive a ring for controlling the spherical power of the lens (6) of variable spherical power to rotate.

2. The visual compensation system as claimed in claim 1, wherein the first optical element (2) and the second optical element (4) are independently rotatable one from the other so that, in at least one position, the resultant cylindrical power generated by the combination of the first optical element (2) and the second optical element (4) has a value lower than 0.1 dioptres.

3. The visual compensation system as claimed in claim 1 or 2, wherein the first optical element (2) and the second optical element (4) are independently rotatable one from the other so that, in at least one position, the resultant cylindrical power generated by the combination of the first optical element (2) and the second optical element (4) has a value of zero.

4. The visual compensation system as claimed in one of claims 1 to 3, wherein the spherical power induced by the combination of the first optical element (2) and the second optical element (4) is at least in part compensated for by the lens (6) of variable spherical power.

5. The visual compensation system as claimed in one of claims 1 to 4, wherein the lens (6) of variable spherical power is a lens containing a fluid and a deformable membrane.

6. The visual compensation system as claimed in one of claims 1 to 4, wherein the lens (6) of variable spherical power is a deformable lens containing a fluid.

7. The visual compensation system as claimed in one of claims 1 to 6, wherein a first mechanism (32, 22) driven by a first motor (42) is designed to rotate the first optical element (2) with a rotary movement centred on the optical axis (X) and wherein a second mechanism (34, 24) driven by a second motor (44) is designed to rotate the second optical element (4) with a rotary movement centred on the optical axis (X).

8. The visual compensation system as claimed in claim 7, wherein a control element (50) is designed to respectively control the first motor (42) and the second motor (44) depending on setpoint data.

9. The visual compensation system as claimed in claim 8, wherein the control element (50) comprises a temperature sensor (62).

10. The visual compensation system as claimed in claim 8 or 9, wherein the control element (50) comprises a sensor (64) of orientation or of movement designed to deliver an orientation datum.

11. The visual compensation system as claimed in claim 10, wherein the control element (50) comprises a computing machine (66) designed to generate control signals depending on at least one of said setpoint data and said orientation datum and to emit control signals respectively addressed to the first motor (42) and to the second motor (44).

12. The visual compensation system as claimed in one of claims 8 to 11, wherein the control element (50) is designed to generate control signals depending on at least one of said setpoint data and a distance between a portion of the system and an eye observing through the system.

13. The visual compensation system as claimed in one of claims 7 to 12, wherein the first mechanism comprises a first cog (22) that interacts with a first worm screw (32) that is securely fastened to a driveshaft of the first motor (42), the first optical element (2) being mounted on the first cog (22), and wherein the second mechanism comprises a second cog (24) that interacts with a second worm screw (34) that is securely fastened to a driveshaft of the second motor (44), the second optical element (4) being mounted on the second cog (24).

14. The visual compensation system as claimed in one of claims 7 to 13, wherein the mechanism designed to drive the controlling ring to rotate comprises a third cog (27) that interacts with a third worm screw (36) that is securely fastened to a driveshaft of said motor (46), the controlling ring being securely fastened to the third cog (27).

15. The visual compensation system as claimed in claim 14 when dependent on claim 13, wherein the first motor (42), the second motor (44) and said motor (46) are placed so as to free a circular geometry over at least 120°, said geometry being centred on the optical axis (X) at a distance smaller than 20 mm from the effective radius of the lenses.

16. The visual compensation system as claimed in one of claims 13 to 15, comprising at least one optical cell (52; 54; 56) associated with one of said cogs (22; 24; 27) so as to determine the position of the associated optical element (2; 4; 6).

17. The visual compensation system as claimed in one of claims 1 to 16, wherein the first optical element, the second optical element and the lens are mounted so as to preserve their respective setpoint positions without supply of electrical power.

18. The visual compensation system as claimed in one of claims 1 to 17, wherein the first optical element (2) and the second optical element (4) are separated by a space of dimension smaller than 1 mm along the optical axis (X).

19. The visual compensation system as claimed in one of claims 1 to 18, wherein the first optical element (2) is a first dioptre formed on a face of a first planar-cylindrical lens and wherein the second optical element (4) is a second dioptre formed on a face of a second planar-cylindrical lens.

20. The visual compensation system as claimed in claim 19, wherein the first optical element (2) is a convex planar-cylindrical lens and wherein the second optical element (4) is a concave planar-cylindrical lens.

21. The visual compensation system as claimed in one of claims 1 to 20, wherein the first optical element (2), the second optical element (4) and the lens (6) are controlled so as to provide a Jackson-cross-cylinder function.

22. The visual compensation system as claimed in one of claims 1 to 21, wherein the lens (6) of variable spherical power is mechanically actuatable so as to make said spherical power vary continuously.

23. An optometric binocular device comprising two optical systems, wherein at least one of the two optical systems is a visual compensation system according to one of claims 1 to 22.

# Fig.1

# Fig.2

# Fig.5

**Fig.3**

**Fig.4**

**EP 3 096 677 B1**

**RÉFÉRENCES CITÉES DANS LA DESCRIPTION**

**Documents brevets cités dans la description**

- US 2004032568 A

- EP 2034338 A